# EUROPEAN PATENT SPECIFICATION

(11) **EP 0 399 482 B1**
(45) Date of publication and mention of the grant of the patent: **04.01.1995**
(21) Application number: 90109734.5
(22) Date of filing: 22.05.1990
(51) Int. Cl.: A61B 5/00, G01N 21/31

(54) **Liver function testing apparatus**
Gerät zur Prüfung der Leberfunktion
Appareil destiné à l'essai du fonctionnement du foie

(30) Priority: 24.05.1989 JP 132345/89
(43) Date of publication of application: 28.11.1990
(73) Proprietor: SUMITOMO ELECTRIC INDUSTRIES, LTD., Osaka-shi, Osaka 541 (JP)
(72) Inventor: Kanda, Masahiko, c/o Osaka Works, Konohana-ku, Osaka (JP)
(74) Representative: Herrmann-Trentepohl, Werner, Dipl.-Ing.

(56) References cited:
- EP-A- 0 298 122
- EP-A- 0 316 745
- EP-A- 0 316 812
- EP-A- 0 359 206

## Description

### BACKGROUND OF THE INVENTION

### Field of the Invention

The present invention relates to a liver function testing apparatus. More specifically, it relates to a liver function testing apparatus for automatically performing measurement for testing and diagnosing a liver function by injecting a specific dye, which is selectively taken in and removed by only the liver, into blood to calculate a value correlating with concentration of the specific dye in the blood.

### Description of the Background Art

In general, the blood plasma disappearance rate and the retention rate for testing and diagnosing of a liver function have been measured by a method of blood collection through use of indocyanine green (hereinafter referred to as ICG) serving as specific dye.

According to this method, intravenous injection of ICG is applied to a testee to perform blood collection three times after lapses of five, ten and 15 minutes from the injection, and blood serum is separated upon coagulation of a blood clot so that absorbance at a wavelength of 805 nm is measured through a spectrophotometer to obtain ICG concentration values in the blood serum after the lapses of five, ten and 15 minutes from a previously obtained calibration curve (corresponding ICG concentration in blood vs absorbance), thereby to calculate the blood plasma disappearance rate and the retention rate.

Japanese Patent Publication No. 60-58649 has proposed a method of measuring the blood plasma disappearance rate and the retention rate without performing blood collection. According to this method, light is applied through the body surface of an organism, which in turn transmits light of a wavelength having high ICG absorption sensitivity and light of a wavelength having substantially no ICG absorption sensitivity. The respective quantities of transmitted light are measured to obtain the blood plasma disappearance rate and the retention rate from time change (dye disappearance curve) of the light quantities.

Japanese Patent Laying-Open No. 64-17630 discloses an apparatus in which light is applied through the body surface of an organism, absorbance of ICG is measured to obtain the blood plasma disappearance rate and the retention rate based on a time change (dye disappearance curve) in the measurement of the absorbance. In this apparatus, calibration is performed before measurement so that changes in blood quantities in the organism can be canceled.

In the conventional method of blood collection, it is necessary to correctly measure the blood collection time after injection. However, the time can not be accurately measured in actual test and the operation for such measurement is complicated. Further, the testee is subjected to heavy mental and physical burdens by blood collection. In addition, the index R_{MAx} measuring method of measuring the blood plasma disappearance rate several times by changing the quantity of ICG injection is often used these days but this method requires blood collection several times and the burdens on the testee are further increased.

According to the measuring method without blood collection as disclosed in Japanese patent Publication No. 60-58649 mentioned above or Japanese Patent Laying-Open No. 61-162934, the output of a sensor actually attached to an organism is fluctuated by influence such as blood flow disturbance caused by oppression on a blood vessel, vibration of the organism subjected to measurement, pulsation in the organism, change of blood volume in the vital tissue (for example, the blood volume being changed by mere vertical movement of an arm) etc., making it difficult to obtain a correct dye disappearance curve.

Consequently, the blood plasma disappearance rate and the retention rate obtained by the dye disappearance curve can not be said to be correct.

The apparatus disclosed in Japanese Patent Laying-Open NO. 64-17630 requires calibration before measurement and therefore the operation is complicated. In addition, the change of blood volume in the vital tissue can be compensated for to some extent but not in a satisfactory manner. This is because the calibration is preferably performed immediately before injection of a specific dye but since the injection of the specific dye takes time in reality, the calibration does not effectively contribute to increase of the measurement precision.

EP-A-0 298 122 discloses a liver function inspection apparatus using a first ray of light having wavelength absorbed by a specific dye and a second ray not absorbed by the dye. After the injection of the dye into the living tissue the concentration of the dye is calculated by means of regression analysis on the basis of the intensities of the light transmitted through the issue. The the dissapearance ratio and the retention ratio of plasma are calculated on the basis of coefficients which have been derived by least square fitting. The known liver function inspection apparatus comprises in particular light source means for exposing vital tissue to first light of a wavelength absorbed by specific dye dosed into blood of said vital tissue to be taken in and removed by the liver and second light of a wavelength not absorbed by said specific dye; photoelectric conversion means for outputting first and second photoelectric conversion signals corresponding to said first light and said second light applied to said vital tissue by said light source means and obtained from said vital tissue; sampling means for sampling first and second photoelectric conversion signals from said photoelectric conversion means; decision means for deciding a coefficient of a regression line expression between the logarithms of the intensities of said first and second photoelectric conversion signals; and arithmetic means for calculating a value correlated with specific dye concentration in said blood on the basis of an output of said sampling means during a prescribed period from the injection of said specific dye and said coefficient of the regression line expression decided by said decision means.

### SUMMARY OF THE INVENTION

Therefore, a principal object of the present invention is to provide a liver function testing apparatus which can remove artifacts such as blood flow disturbance, vibration, or pulsation in an organism and change of blood volume in the vital tissue when a sensor is attached to the organism and which enables correct measurement.

Briefly stated, in the apparatus of the present invention, vital tissue is exposed to first light of a wavelength absorbed by specific dye which is dosed into blood of the vital tissue to be taken in and removed by the liver and second light of a wavelength not absorbed by the dye. First and second photoelectric conversion signals corresponding to the first light and the second light obtained from the vital tissue are sampled and only pulsation components are detected. The regression coefficient of a linear regression expression between the logarithms of the pulsation components of the first and second photoelectric conversion signals immediately before injection of the specific dye, and a base value using the sampled first and second photoelectric conversion signals are determined. A value correlated with specific dye concentration in the blood is calculated based on a sampling signal during a prescribed period from the injection of the specific dye and the determined coefficient and base value.

Therefore, according to the present invention, there is no necessity of collecting blood and the testee needs only to be subjected to injection of specific dye, which makes it possible to considerably reduce the mental and physical burdens on the testee. Furthermore, it is possible to remove artifacts such as blood flow disturbance, vibration or pulsation in an organism to which a sensor is attached.

In a preferred embodiment of the present invention, first and second photoelectric conversion signals are detected several times and if intensities of pulsation components of the detected first and second photoelectric conversion signals are represented as T_{1P} and T₂p, a linear regression analysis is performed according to the following equation:
log T_{1P} = A. log T_{2P},

whereby a constant A is obtained. If the sampled first and second photoelectric conversion signals are represented as T_{1c} and T₂α, the base value So' is obtained by the equation:

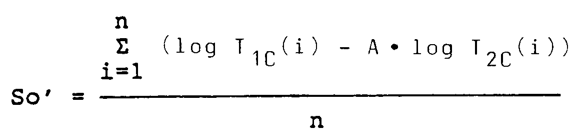

At the same time, the maximum value of the first photoelectric conversion signal sampled several times is obtained as T₁₀.

According to another preferred embodiment of the invention, if the values of the sampled first and second photoelectric conversion signals are represented as T₁ and T₂, the value Cg correlated with the specific dye concentration is calculated by the below indicated equation based on the constant A, base value So' and maximum value T₁₀ obtained as described above.

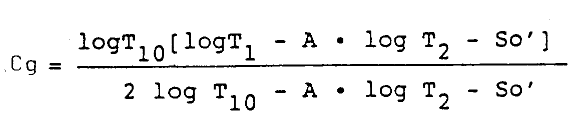

In this preferred embodiment of the invention, the blood plasma disappearance rate of the specific dye or at the same time the retention rate in a prescribed period of the specific dye can be obtained based on a calculated coefficient of a simulation function.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Figs. 1 to 4 are diagrams for explaining the principle of the present invention.
Fig. 5 is a schematic block diagram showing an embodiment of the present invention.
Fig. 6 is a timing chart for detecting light quantities of wavelengths λ₁ and X₂ after passage through a prescribed optical path in a tested object.
Fig. 7 is a diagram showing data stored in a RAM as shown in Fig. 5.
Figs. 8A to 8C are flow charts for explaining a specified operation of the embodiment of the present invention, in which Fig. 8A shows a data sampling subroutine, Fig. 8B shows a calibration mode, and Fig. 8C shows a measurement mode.
Figs. 9 to 12 illustrate exemplary displays on a display part as shown in Fig. 5.
Fig. 13 shows an example of a disappearance curve of specific dye measured by the present invention.

### DESCRIPTION OF THE PREFERRED EMBODIMENTS

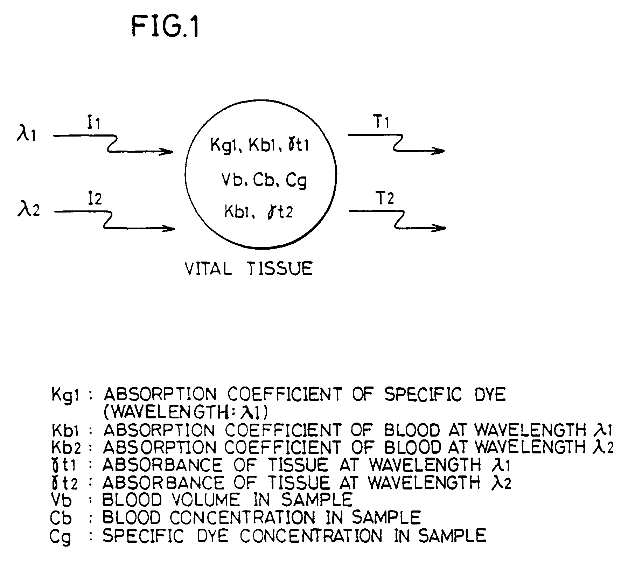

Figs. 1 to 4 are diagrams for illustrating the principle of biocalibration in the present invention.

In the following description, symbols I₁ and 1₂ indicate quantities of light having a wavelength λ₁ which is largely absorbed by specific dye and light of a wavelength X₂ which is not absorbed by the specific dye incident upon vital tissue, and symbols T₁ and T₂ indicate light quantities after passage through a prescribed optical path in the vital tissue. Relations between the incident light quantities I₁ and 1₂ and the passing light quantities T₁ and T₂ in injection of the specific dye are as follows: 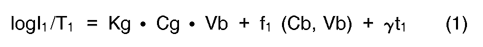 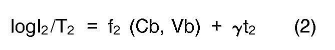 where kg represents an absorption coefficient of the specific dye at wavelength λ₁ ; γt2 represents absorbance of tissue at wavelength λ2; vb represents a blood volume in sample; Cb represents a blood concentration in sample; and Cg represents a specific dye concentration. Symbols f₁ and f₂ represent functions which are determined by characteristics of blood at the wavelengths λ₁, and x₂.

On the other hand, relations between the incident light quantities I₁ and 1₂ and the passing light quantities T₁ and T₂ before injection of the specific dye are as follows:

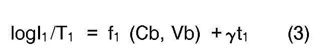

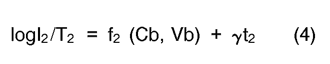

If only the pulsation components of the passing light quantities T₁ and T₂ are detected, the above indicated equations (3) and (4) become as follows:

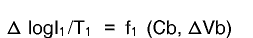

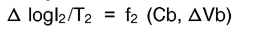

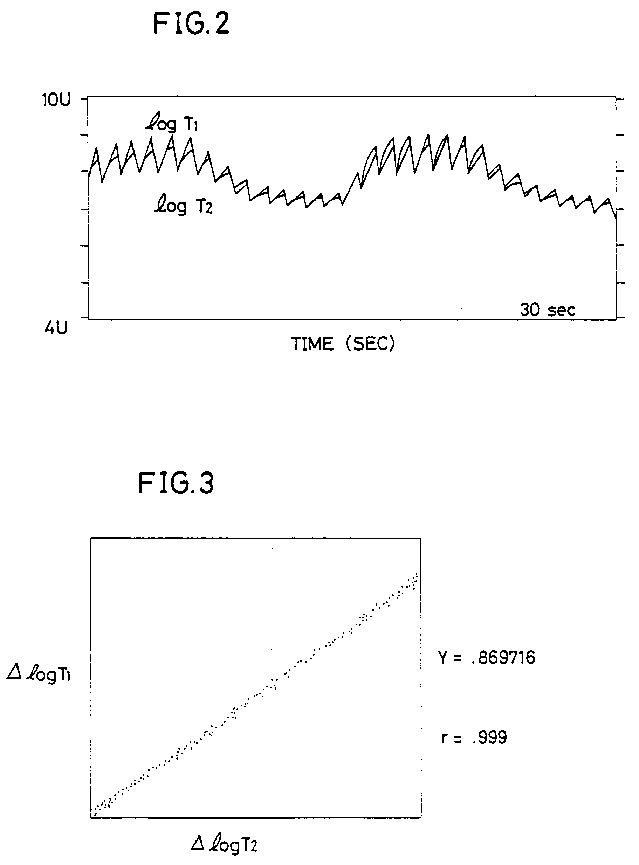

This relation is measured as shown in Fig. 2, to be in the linear relation as shown in Fig. 3. This is the data in case of attaching a sensor to an organism and fluctuating the blood volume in the organism. It has been confirmed that such linearity has reproducibility, with no individual difference.

Therefore, the expressions (3) and (4) would appear as follows:

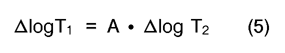

Thus, it is expressed as follows:

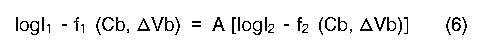

Since the main component changing in the expressions (3) and (4) before injection of the specific dye is the blood, the expression (6) can be represented as follows:

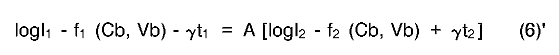

Then, by using the expressions (1) and (2) after injection of the specific dye to obtain 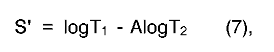 the following expression is obtained:

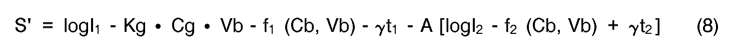

If the expression (6) is used in the above indicated expression (8), the following expression is obtained:

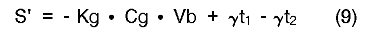

In addition, if the value So' before injection of the specific dye is represented as: So' = γt1 - γt2 and a relation of S' - So' is set, the function S is expressed as follows:

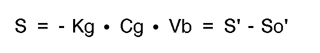

In consequence, it is understood that a signal of the function S can be obtained by using the diagram of Fig. 3 as a biocalibration curve.

As to the function S, although the absorption coefficient Kg is constant, it can be considered that the blood volume Vb is changed from time to time, and an accurate value of the specific dye concentration can not be obtained, because the value of Cg is influenced by the blood volume Vb in the organism.

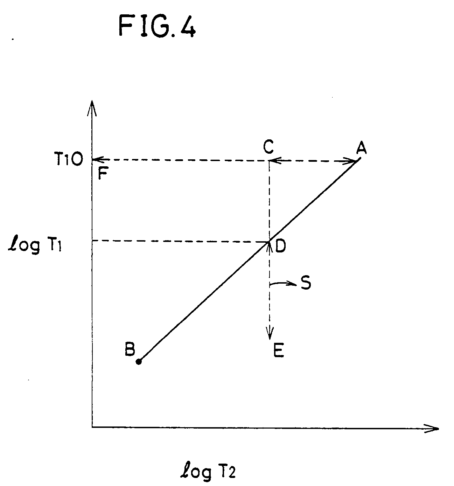

Referring to Fig. 4, AB represents a calibration curve. When the specific dye is injected, a signal of only logT₁ is fluctuated, to reach a point E, for example. At this time, DE which is the value of the function S after a lapse of t₁ minutes becomes the function S as shown in the expression (9). The blood volume Vb in the expression (9) can be interpreted as CD, and hence, normalizing the Y coordinate of a point A as Tio, the same is expressed as follows:

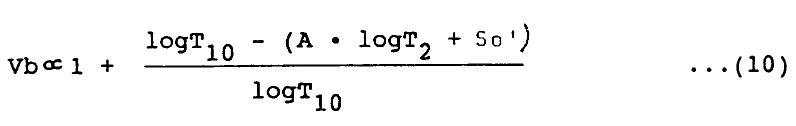

Hence, a signal Cg corresponding to the specific dye concentration Cg can be found by the expressions (7) and (10) as follows:

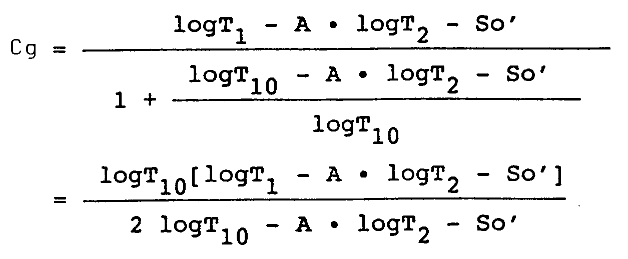

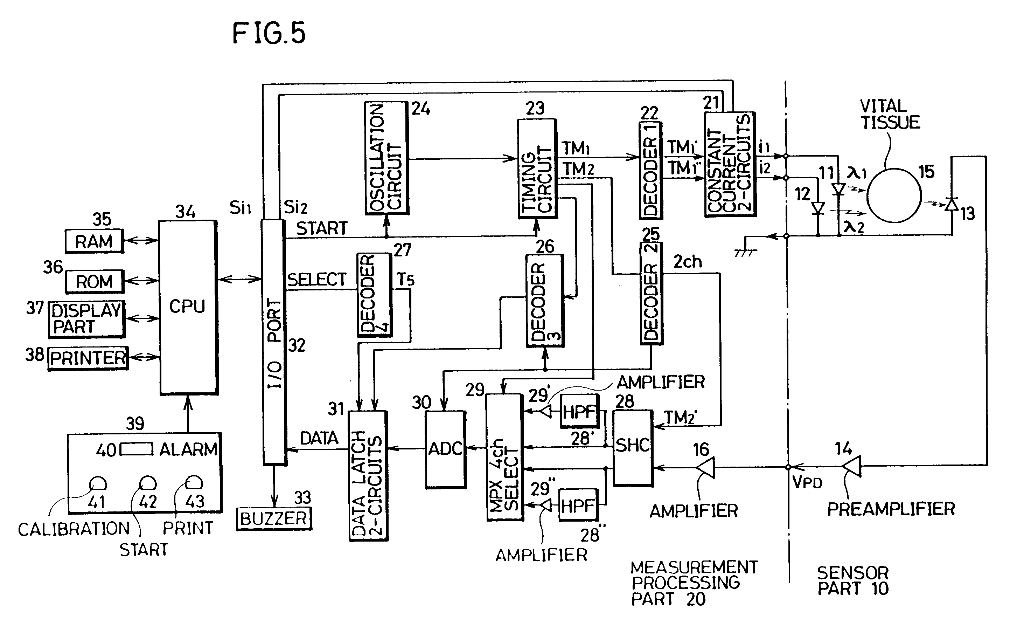

Fig. 5 is a schematic block diagram showing an embodiment of the present invention.

Referring to Fig. 5, a liver function testing apparatus is formed by a sensor part 10 and a measurement processing part 20. The sensor part 10 includes a first light source 11, a second light source 12, a light receiving element 13 and a preamplifier 14.

The first light source 11 and the second light source 12 generate optical pulses of a wavelength À₁ having large absorbance to specific dye and optical pulses of a wavelength X₂ having no absorbance, respectively. The light receiving element 13 receives light applied to vital tissue 15 from the light sources 11 and 12 to pass through a prescribed optical path. The light sources 11 and 12 are driven by the measurement processing part 20 to alternately emit light by pulse operation, respectively.

The measurement processing part 20 includes a CPU 34 which operates as arithmetic means. The CPU 34 supplies a start signal to an oscillation circuit 24 and a timing circuit 23 through an I/O port 32. The oscillation circuit 24 regularly oscillates to produce a prescribed clock signal.

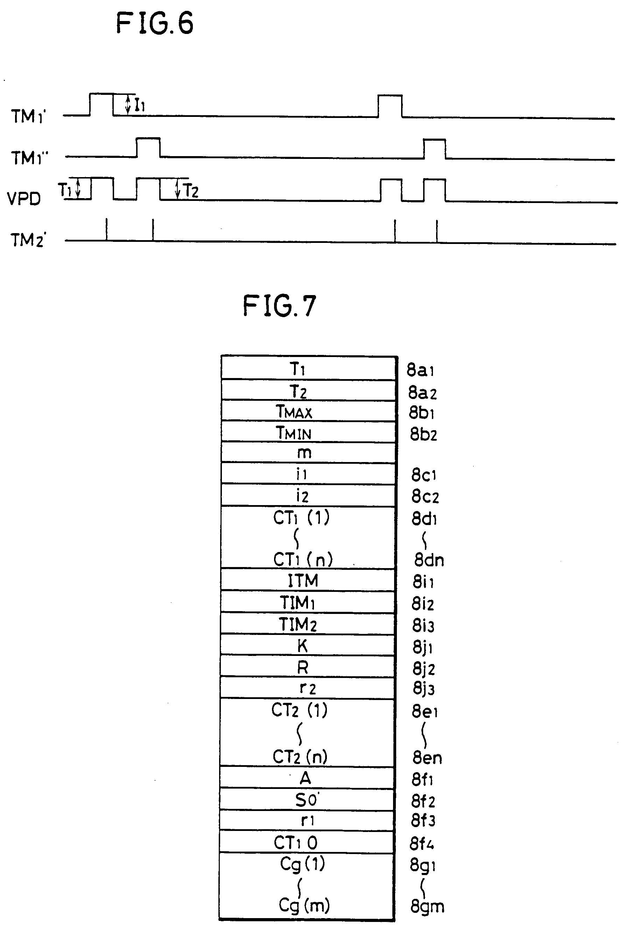

This clock signal and the aforementioned start signal are utilized to supply constant currents ii and i₂ to the first light source 11 and the second light source 12 from a constant current circuit 21 through the timing circuit 23 and a decoder 22 at timings TM₁' and TM₁" in Fig. 6.

The light emitted from the first light source 11 and the light emitted from the second light source 12 pass through the prescribed optical path in the vital tissue 15, to be incident upon the light receiving element 13. A current generated from the light receiving element 13 is supplied to the preamplifier 14 and it is subjected to current-to-voltage conversion, while being amplified.

An output signal of the preamplifier 14 is amplified to a level within a prescribed range by an amplifier 16 provided in the measurement processing part 20, whereby output such as V_{f}α in Fig. 6 is obtained. A sample-and-hold circuit 28 samples and holds the output at timing TM₂', shown in Fig. 6, generated by the timing circuit 23 and a decoder 25.

The signal thus sampled and held is selected by a multiplexer 29 with voltages T₁ and T₂ shown in Fig. 6 being maintained and the selected signal is converted into a digital signal by an A-D converter 30, to be latched by a data latch 31. At this time, the multiplexer 29, the A-D converter 30 and the data latch 31 are controlled in timing by the timing circuit 23 and the decoder 26.

The latched data are timed by a decoder 27 by a select signal outputted from the CPU 34 through the I/O port 32, to be taken in a RAM 35 as digital signals T₁ and T₂. The I/O port 32 is connected with a buzzer 33, which informs timing for injecting the specific dye. Further, the CPU 34 is connected with the RAM 35, a ROM 36, a display part 37 and an operation part 28.

A slow component is removed from the sampled and held signal by using a high-bandpass filter HPF and an amplifier as shown in Fig. 5, whereby only the pulsation component is taken out. This operation is performed for both of T₁ and T₂ and the respective outputs are amplified by the amplifier 16 and taken in the RAM 35 as digital signals as described above by means of the multiplexer 29.

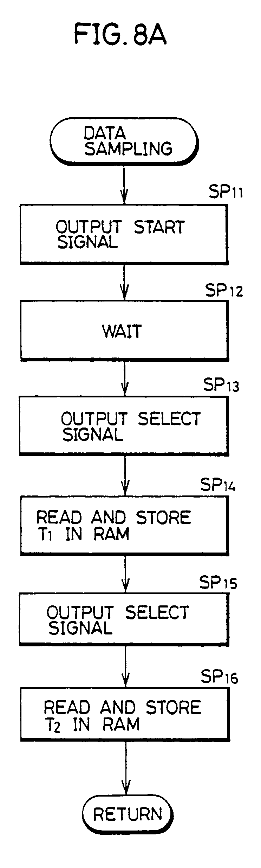

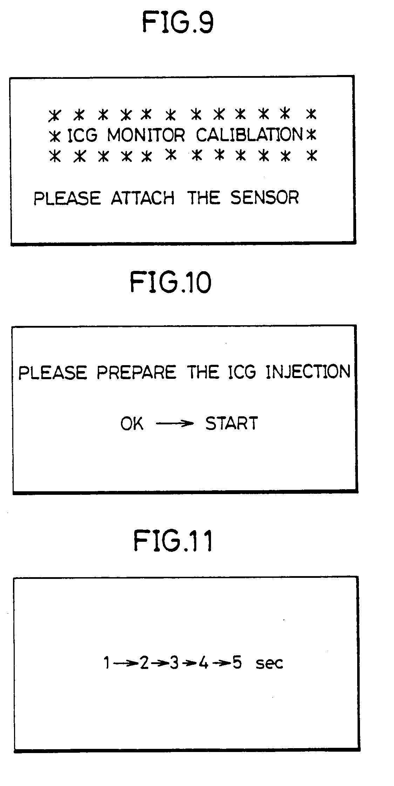

The RAM 35 stores data as shown in Fig. 7 as hereinafter described, and the ROM 36 stores programs based on flow charts as shown in Figs. 8A to 8C as hereinafter described. The display part 37 displays data as shown in Figs. 9 to 12, as hereinafter described. A printer 38 prints the result of a liver function test.

The function part 39 includes an alarm LED 40, a calibration key 41, a start key 42 and a print key 43. The alarm LED 40 displays an alarm when reliability of the test result is small. The calibration key 41 is used to set a biocalibration mode. The start key 42 is used to command start of a measurement mode and the print key 43 is used to command printout of the test result.

Fig. 7 illustrates data stored in the RAM 35 as shown in Fig. 5 and Figs. 8A to 8C are flow charts for illustrating an operation of the embodiment of the present invention. Figs. 9 to 12 are illustrative of exemplary displays on the display part as shown in Fig. 5.

With reference to Figs. 5, 8A to 8C and 13, description is now made of the operation of the embodiment of the present invention.

First of all, steps SP11 to SP16 shown in Fig. 8A are employed to sample quantities of light of a pair of wavelengths λ1 and λ2 after passage through a measured object and to store the same in the RAM 35. More specifically, the CPU 34 outputs the start signal from a line as shown in Fig. 5 through the I/O port 23 in step SP11. The voltages T₁ and T₂ are latched by the start signal, as hereinabove described. The CPU 34 waits until the data are latched in step SP12.

Then, in step SP13, the CPU 34 outputs the select signal to a select line as shown in Fig. 5 through the I/O port 32, to read the data of T₁ through the I/O port 32 in step SP14, thereby to store the same in a storage area 8a1 of the RAM 35 as shown in Fig. 6.

Similarly, the CPU 34 stores the data of T₂ in a storage area 8a2 of the RAM 35 in steps SP15 and SP16.

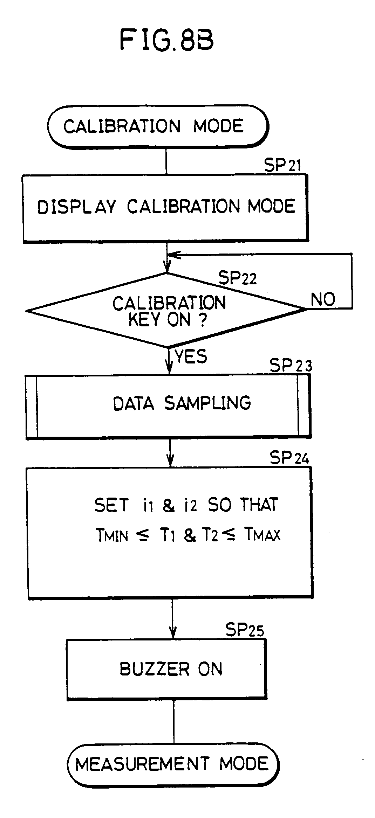

Upon completion of the aforementioned operation in step SP16, the CPU 36 returns to the original step. This will be described with reference to Fig. 8B showing the biocalibration mode.

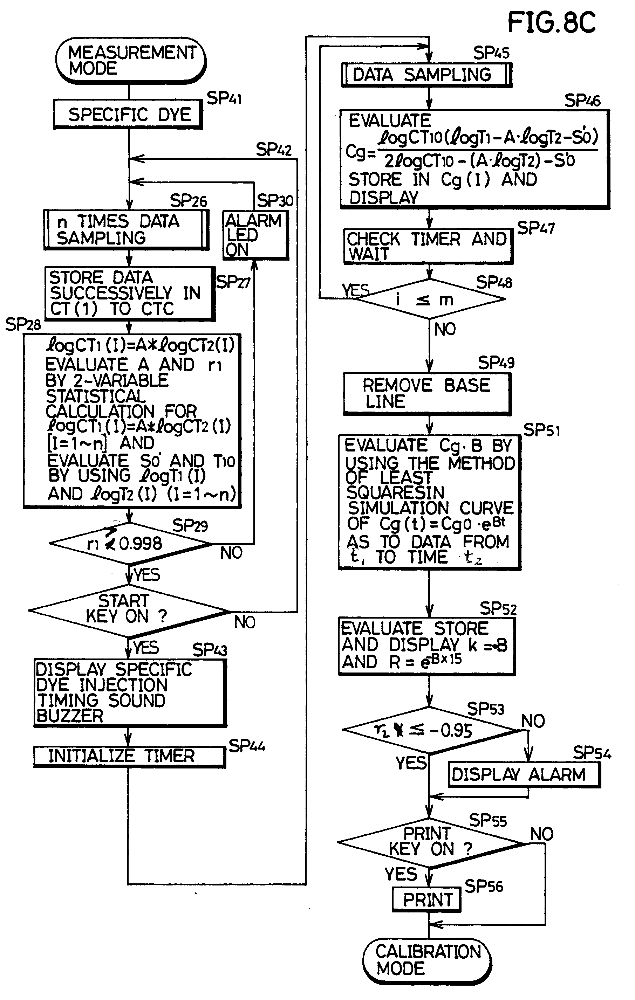

The biocalibration mode is started before operation of a measurement mode shown in Fig. 8C as hereinafter described. In step SP21, the CPU 34 displays the biocalibration mode on the display part 37. This display indicates that the apparatus enters the biocalibration mode and indicates mounting of the sensor part 10 as shown in Fig. 9, for example. In accordance with this indication, an operator attaches the sensor part 10 to the object 13 to be tested.

Thereafter the CPU 34 waits until the calibration key 41 is operated in step SP22. When the calibration key 41 is operated, the CPU 34 advances to step SP23, to execute the data sampling subroutine as shown in Fig. 8A, as hereinabove described.

Then, the CPU 34 controls the constant current circuit 21 by using lines Si1 and Si2 as shown in Fig. 5 so that the data T₁ and T₂ read in step SP23 are within ranges of light quantity data T_{MAx} and T_{MIN} stored in storage areas 8bl and 8b2 of the RAM 35. The CPU 34 then stores set values of currents of the lines Si1 and Si2 in storage areas 8c1 and 8c2 in the RAM 35. Thereafter the currents of the lines Si1 and Si2 regularly flow to the light sources 11 and 12.

Then, the CPU 34 sounds the buzzer in step SP25, to inform that power setting is completed. Then, it proceeds to the measurement mode.

Next, the measurement mode will be described with reference to Fig. 8C.

Steps SP26 to SP29 in Fig. 8C are shown as a flow chart for performing the above mentioned biocalibration. More specifically, the values of CT₁ and CT₂ converted as the pulsation signals in steps SP26 and SP27 are sampled n times, so that CT1 (1) to CT₁(n) are stored in the areas 8d1 to 8dn of the RAM 35 and CT₂(1) to CT₂(n) are stored in the areas 8e1 to 8en.

In the subsequent step SP28, the CPU 34 performs 2-variable statistical calculation with respect to logCT₁ (I) and logCT₂(1) (I = 1 to n) in the following manner:

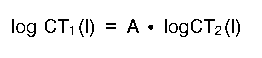

Then, using the value A thus obtained, a correlation coefficient r₁ and the n values of T₁(I) and T₂(1) calculated at the same time, the value So' is calculated by the following expression: 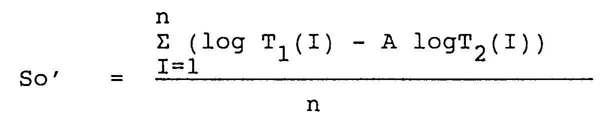 and the maximum value T₁ of T₁ (I) (I = 1 to n) is calculated, whereby the values thus obtained are stored in the areas 8f1, 8f2, 8f3 and 8f4 of the RAM 35. Then, in step SP29, the CPU 34 determines whether the correlation coefficient r₁ is at least 0.998 in order to verify reliability of the biocalibration. If it is less than 0.998, the CPU 34 advances to step SP30 to light the alarm LED 40 and returns to step SP22 to perform again biocalibration. On the other hand, if the correlation coefficient r₁ is 0.998 or more, the CPU 34 proceeds to the measurement mode shown in Fig. 8C. The reference value 0.998 of the correlation coefficient r₁ herein employed is a mere example, which is determined by performance of the entire apparatus. In step SP43, a display for injection of the specific dye is made on the display part 37.

This display is made for example to indicate injection of a specific dye, e.g., ICG. In accordance with the display, the operator prepares for injection of the specific dye to the testee. In step SP42, the CPU 34 waits until the start key 42 is operated. During this waiting, the operation in steps SP26 to SP29, namely, the biocalibration is performed repeatedly and the calibration values (A, So' and Tᵢₒ) immediately before depression of the start key 42 are stored in the areas 8f1 to 8f4 in the RAM 35.

When the start key 42 is depressed, the calibration data immediately before the depression are employed for the subsequent measurement.

Upon determination that the start key 42 is operated, the CPU 34 displays timing for injecting the specific dye in step SP43, while sounding the buzzer 33.

This is displayed as 1 → 2 → 3 → 4 → 5 as shown in in Fig. 11, for example, so that the operator injects the specific dye upon display of "5". The CPU 34 generates a first sound from the buzzer 33 with the displays of "1 ", "2", "3" and "4", while generating a different sound from the buzzer 33 upon display of "5".

Upon generation of the sound and display, the operator injects the specific dye. The CPU 34 sets "0" as the initial value of a timer in step SP44.

In step SP45, the CPU 34 executes a data sampling program, which is the subroutine as hereinabove described with reference to Fig. 8A. Then, the sampling data are stored in the areas 8a1 to 8a2 of the RAM 35 as T₁ to T₂, respectively. In step SP46, the CPU 34 performs operation based on the following operation expression by using the coefficients A, So', and T₁₀ stored in the areas 8f1, 8f2 and 8f4 of the RAM 35 in the biocalibration mode as hereinabove described with reference to Fig. 8B, to store Cg(l) in an area 8g1 of the RAM 35:

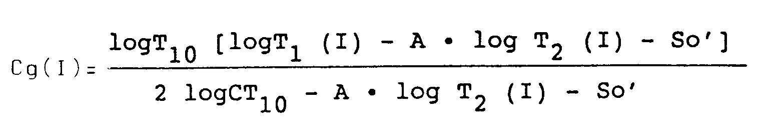

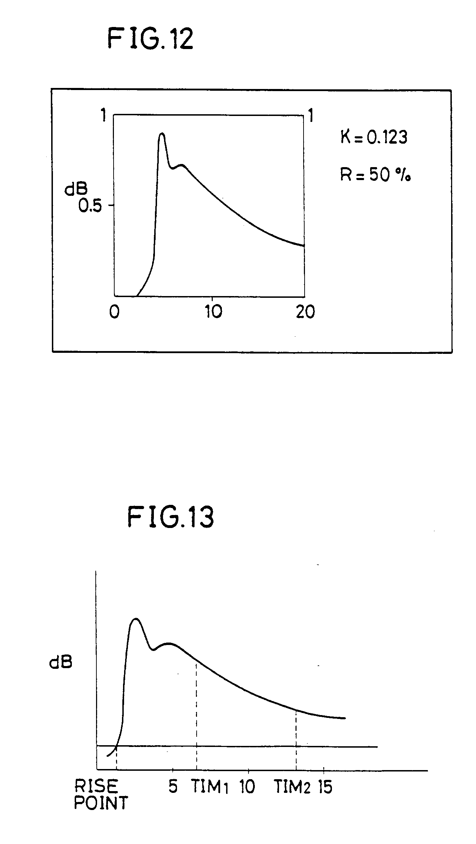

The value of Cg(l) is displayed on the display part 37 in step SP46 in a mode as shown in Fig. 12, for example. Referring to Fig. 12, the abscissa indicates the elapsed time from injection of the specific dye and the ordinate indicates the value of Cg(I). If m represents the sampling number of a disappearance curve of the specific dye, symbol I indicates integers 1 to m. If tₛ represents a measuring time of the disappearance curve, a single sampling time is ITM = tₛ/ (m - 1). The same coincides with the injection time of the specific dye in the case of I = 1, as a matter of course. In step SP47, the CPU 34 waits during this sampling ITM.

Upon a lapse of this standby time, the CPU 34 determines whether or not i is greater than m in step SP48. The CPU 34 advances to step SP49 if i is greater than m while the same again returns to step SP45 to repeat sampling if the former is less than the latter. The data Cg(l) stored in the areas 8g1 to 8gm of the RAM 35 draw a disappearance curve of the specific dye as shown in Fig. 13, for example, and the leading edge thereof is detected so that the preceding data are subtracted as baselines from the respective values of Cg(I), to be again stored in the areas 8g1 go 8gm. Needless to say, T₁ to T₂ in step SP45 may be average values of k times, in order to to improve the accuracy of measurement.

Then, in step SP51, the CPU 34 finds the constants A and B by using the method of least squares in a simulation curve of: 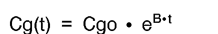 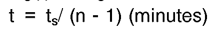 with respect to data between times t₁ to t₂ (0 < t₁ < t₂ < tₛ) within the data Cg(l) stored in the areas 8g1 to 8gm.

Then, the CPU 34 performs calculation of the blood plasma disappearance rate k = -B and the t-minute (t = 15) retention rate 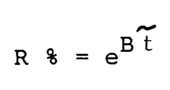 in step SP52, to evaluate k and R. The values k and R thus evaluated are stored in areas 8jl and 8j2 of the RAM 35, respectively. At this time, the CPU 34 evaluates a correlation coefficient r₂ by the method of least squares, to make the obtained correlation coefficient r₂ stored in a storage area 8j3 of the RAM 35. The CPU 34 further generates an end sound from the buzzer 14.

Further, the CPU 34 makes the values k and R appear on the display part 26 in a mode as shown in Fig. 12, for example. Then, in step SP53, the CPU 34 determines whether or not the correlation coefficient r₂ is less than -0.95, for example. This determination is made to check the degree of correlation since correlation is improved as the correlation coefficient r₂ approaches -1. The value -0.95 is provisionally selected between zero and -1, and reliability of the apparatus is improved as the value comes closer to -1.

If the correlation coefficient r₂ is greater than -0.95, for example, the CPU 34 determines that reliability is insufficient and lights the alarm LED 40 in step SP54. On the other hand, if the correlation coefficient r₂ is less than -0.95, for example, in step SP53, the CPU 34 advances to step SP55 without flashing the alarm LED 281, since the measurement is reliable.

In step SP55, the CPU 34 determines whether or not the print key 43 is operated, to make the printer 38 print the values k and R % if the determination is of YES.

If necessary, the CPU 34 also makes characteristic dye disappearance curves of Cg(l) stored in the areas 8gl to 8gn of the RAM 35 printed, to advance to the biocalibration mode as shown in Fig. 8B.

Also when a determination is made that the print key 43 is not operated in step SP55, the CPU 34 advances to the calibration mode.

By using the value k obtained according to the present invention, the apparatus of the present invention is applicable to an apparatus for measuring R_{MAX} by calculating the value k for various amounts of ICG injection.

As described in the foregoing, according to the embodiment of the present invention, vital tissue is exposed to optical pulses of a wavelength considerably absorbed by a specific dye and optical pulses of a wavelength not absorbed by the dye at prescribed levels and the optical pulses transmitted through a prescribed optical path in the vital tissue are detected so that biocalibration is performed based on the detection output. Thus, using the coefficient thus obtained, the blood plasma disappearance rate and the retention rate of the specific dye after injection thereof are obtained according to a prescribed calculation expression based on an output of received light in a prescribed period from the injection. Consequently, it is possible to correctly control the time related with the disappearance curve of the specific dye and to obtain correct data.

In addition, the present invention makes it possible to obtain the blood plasma disappearance rate and the retention rate from many data of disappearance curves, not from several samples as in the conventional blood collection method.

In addition, the measurement method can be simplified compared with the conventional testing method in which the quantity of ICG injection is changed and measured several times to obtain the blood plasma disappearance rate and the retention rate.

Furthermore, the present invention makes it possible to remove artifacts such as blood flow disturbance, vibration and pulsation of an organism and change in blood volume in the organism in attachment of a sensor to the organism, which are problems in the prior art, and thus correct measurement can be made. Since calibration is performed immediately before measurement, the precision of measurement is further improved. Accordingly, the apparatus of the present invention can be effectively utilized in the technical fields of measurement of specific dye in vital tissue in a non-invasive manner.

## Claims

1. A liver function apparatus for testing liver function, comprising:
light source means (11, 12) for exposing vital tissue to first light of a wavelength absorbed by specific dye dosed into blood of said vital tissue to be taken in and removed by the liver and second light of a wavelength not absorbed by said specific dye;
photoelectric conversion means (13) for outputting first and second photoelectric conversion signals corresponding to said first light and said second light applied to said vital tissue by said light source means and obtained from said vital tissue;
sampling means (28) for sampling first and second photoelectric conversion signals from said photoelectric conversion means;
pulsation detecting means (28', 28", SP51-SP54) for detecting only pulsation components of said first and second photoelectric conversion signals from said photoelectric conversion means;
decision means (34, SP28) for deciding the regression coefficient of a regression line between the logarithms of the intensities of said pulsation components of said first and second photoelectric conversion signals obtained from said pulsation detecting means immediately before injection of said specific dye and a base value (So') using said first and second photoelectric conversion signals sampled by said sampling means; and
arithmetic means (34) for calculating a value (Cg) correlated with specific dye concentration in said blood on the basis of an output of said sampling means during a prescribed period from the injection of said specific dye and said slope of the regression line and said base value (So') decided by said decision means.

2. A liver function testing apparatus in accordance with claim 1, wherein
said pulsation detecting means (28', 28", SP51-SP54) includes means for sampling said first and second photoelectric conversion signals a plurality of times, and
said decision means (34, SP28) for deciding said base value (So') by using said regression coefficient of said regression line expression includes means for obtaining a constant A by performing regression line analysis in accordance with the following operation expression: 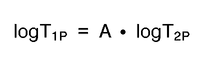 where T_{1P} and T₂p represent average values of said first and second photoelectric conversion signals sampled by said sampling means (28) a plurality of times.

3. A liver function testing apparatus in accordance with claim 1, wherein
said sampling means (28) includes means for sampling said first and second photoelectric conversion signals a plurality of times, and means for obtaining said base value So' according to the following operation expression: 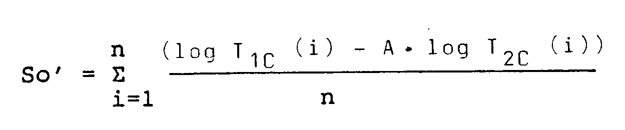 where T_{ic} and T_{2c} represent said first and second photoelectric conversion signals sampled by said plurality of times (n times), and obtaining a maximum value T₁₀ of said first photoelectric conversion signal.

4. A liver function testing apparatus in accordance with claim 1, further including coefficient calculating means (34, SP52) for obtaining a coefficient (B) of a simulation function as a function time by using the method of least squares on the basis of said value (Cg) correlated with said specific dye concentration obtained by said arithmetic means.

5. A liver function testing apparatus in accordance with claim 4, further including means (34, SP52) for obtaining a blood plasma disappearance rate (k=-B) of said specific dye on the basis of said coefficient (B) of said simulation function obtained by said coefficient calculating means.

6. A liver function testing apparatus in accordance with claim 5, further including means (37, 38) for outputting said blood plasma disappearance rate (K) obtained by said means for obtaining said blood plasma disappearance rate.

7. A liver function testing apparatus in accordance with claim 4, further including means (34, SP52) for obtaining a retention rate (R) of said specific dye in said prescribed time on the basis of said coefficient of said simulation function obtained by said coefficient calculating means.

8. A liver function testing apparatus in accordance with claim 7, further including means (37, 38) for outputting said retention rate (R) obtained by said means for obtaining said retention rate.

9. A liver function testing apparatus in accordance with claim 2, wherein
said arithmetic means (34) includes means for calculating said value Cg correlated with said specific dye concentration on the basis of said constant A obtained by said obtaining means in accordance with the following operation expression: 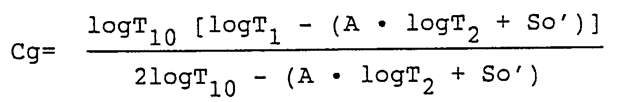 where T₁ and T₂ represent values of said sampled first and second photoelectric conversion signals.

10. A liver function testing apparatus in accordance with claim 4, wherein
said coefficient calculating means includes means for calculating constants A and B on the basis of the following operation expression: 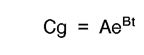 where t represents said prescribed period after injection of said specific dye.

11. A liver function testing apparatus in accordance with claim 5, wherein
said means for obtaining said blood plasma disappearance rate includes means for performing calculation of the following operation expression: 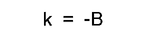 where k represents said blood plasma disappearance rate.

12. A liver function testing apparatus in accordance with claim 7, wherein
said means for obtaining said retention rate includes means for performing calculation of the following operation expression: 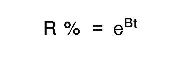 where R % represents said retention rate.

13. A liver function testing apparatus in accordance with claim 1, wherein
said decision means includes means (SP28) for calculating a correlation coefficient r₁ of said regression line expression.

14. A liver function testing apparatus in accordance with claim 12, further including informing means (33, SP30) for giving an alarm when said correlation coefficient (ri) obtained by said means for calculating said correlation coefficient is less than a predetermined value.

15. A liver function testing apparatus in accordance with claim 4, wherein
said coefficient calculating means includes means (SP52) for calculating a correlation coefficient (r₂) of said simulation function.

16. A liver function testing apparatus in accordance with claim 15, further including informing means (33, SP54) for giving an alarm when said correlation coefficient (r₂) of said simulation function is greater than a predetermined value.

17. A liver function testing apparatus in accordance with claim 1, further including mode selection means (41, 42) for selecting a biocalibration mode for performing operation for deciding said regression coefficient of said regression line expression by said decision means and a measurement mode for performing operation for calculating said value (Cg) correlated with said specific dye concentration by said arithmetic means.

18. A liver function testing apparatus in accordance with claim 17, further including means (42) for activating said decision means in response to selection of said biocalibration mode by said mode selection means.

19. A liver function testing apparatus in accordance with claim 17, further including means for activating said arithmetic means (34) in response to selection of said measurement mode by said mode selection means (41, 42).

20. A liver function testing apparatus in accordance with claim 1, further including setting means (SP241-SP249) for setting intensity levels of said first light and said second light emitted from said light source means so that levels of said first and second photoelectric conversion signals are within a predetermined range.

## Patentansprüche

1. Gerät zur Prüfung der Leberfunktion, umfassend:
eine Lichtquelleneinrichtung (11, 12), um eines lebendes Gewebe mit einem ersten Licht einer Wellenlänge, die einen bestimmten Farbstoff absorbiert, der in das Blut des lebenden Gewebes eingegeben wurde, um von der Leber eingenommen und entfernt zu werden, und mit einem zweiten Licht einer Wellenlänge auszusetzen, die von dem bestimmten Farbstoff nicht absorbiert wird;
eine photoelektrische Konversionseinrichtung (13) zum Ausgeben eines ersten und zweiten photoelektrischen Konversionssignales entsprechend dem ersten und zweiten Licht, das dem lebenden Gewebe durch die Lichtquelleneinrichtung zugeführt wurde und von dem lebenden Gewebe erhalten wird;
eine Abtasteinrichtung (28) zum Abtasten der ersten und zweiten photoelektrischen Konversionssignale von der photoelektrischen Konversionseinrichtung;
eine Pulsationsdetektoreinrichtung (28', 28", SP51-SP54) zum Detektieren von nur Pulsationskomponenten der ersten und zweiten photoelektrischen Konversionssignale von der photoelektrischen Konversionseinrichtung;
eine Entscheidungseinrichtung (34, SP28) zum Bestimmen des Regressionskoeffizienten einer Regressionslinie zwischen dem Logarithmus der Intensitäten der Pulsationskomponenten der ersten und zweiten photoelektrischen Konversionssignale, welche von der Pulsationsdetektoreinrichtung unmittelbar vor Einspritzen des bestimmten Farbstoffes erhalten werden, und eines Basiswertes (So'), welcher die ersten und zweiten photoelektrischen Konversionssignale verwendet, die von der Abtasteinrichtung abgetastet wurden; und
eine Arithmetikeinrichtung (34) zum Berechnen eines Wertes (Cg), welcher mit der Konzentration des bestimmten Farbstoffes in dem Blut korreliert ist, auf der Basis eines Ausgangs der Abtasteinrichtung während einer vorgegebenen Zeitdauer ab der Einspritzung des bestimmten Farbstoffes und der Steigung der Regressionslinie und des Basiswertes (So'), welcher von der Entscheidungseinrichtung bestimmt wurde.

2. Gerät zur Prüfung der Leberfunktion nach Anspruch 1, worin die Pulsationsdetektoreinrichtung (28', 28", SP51-SP54) eine Einrichtung zum mehrfachen Abtasten der ersten und zweiten photoelektrischen Konversionssignale einschließt und
die Entscheidungseinrichtung (34, SP28) zum Bestimmen des Basiswertes (So') durch Verwendung der Regressionskoeffizienten der Regressionslinie eine Einrichtung zum Erhalten einer Konstante A durch Ausführen einer Regressionslinienanalyse in Übereinstimmung mit dem folgenden Ausdruck umfaßt: 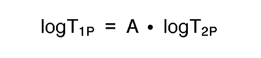 wobei Tₗp und T_{2P} Mittelwerte der ersten und zweiten photoelektrischen Konversionssignale darstellen, die von der Abtasteinrichtung (28) mehrmals abgetastet wurden.

3. Gerät zur Prüfung der Leberfunktion nach Anspruch 1, worin die Abtasteinrichtung (28) eine Einrichtung zum mehrfachen Abtasten der ersten und zweiten photoelektrischen Konversionssignale und eine Einrichtung zum Erhalten des Basiswertes So' gemäß dem folgenden Ausdruck umfaßt: 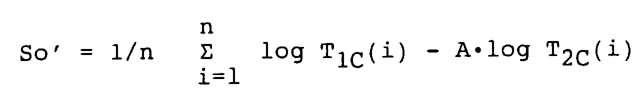 wobei T_{1c} und T_{2c} die ersten und zweiten photoelektrischen Konversionssignale darstellen, welche mehrfach (n-Male) abgetastet wurden, und wobei ein Maximumswert T₁₀ des ersten photoelektrischen Konversionssignales erhalten wird.

4. Gerät zur Prüfung der Leberfunktion nach Anspruch 1, das weiterhin eine Koeffizientenberechnungseinrichtung (34, SP52) umfaßt zum Erhalten eines Koeffizienten (B) einer Simulationsfunktion als eine Funktion der Zeit unter Verwendung der Methode der kleinsten Quadrate auf der Basis des Wertes (Cg), welcher mit der Konzentration des bestimmten Farbstoffes korreliert ist und von der Arithmetikeinrichtung erhalten wurde.

5. Gerät zur Prüfung der Leberfunktion nach Anspruch 4, das weiterhin eine Einrichtung (34, SP52) umfaßt zum Erhalten einer Blutplasmaschwundrate (K = - B) des bestimmten Farbstoffes auf der Basis des Koeffizienten (B) der Simulationsfunktion, welcher durch die Koeffizientenberechnungseinrichtung erhalten wurde.

6. Gerät zur Prüfung der Leberfunktion nach Anspruch 5, weiterhin umfassend eine Einrichtung (37, 38) zum Ausgeben der Blutplasmaschwundrate (K), welche durch die Einrichtung zum Erhalten der Blutplasmaschwundrate erhalten wurde.

7. Gerät zur Prüfung der Leberfunktion nach Anspruch 4, weiterhin umfassend eine Einrichtung (34, SP52) zum Erhalten einer Rückhalterate (R) des bestimmten Farbstoffes in einer vorgegebenen Zeit auf der Basis des Koeffizienten der Simulationsfunktion, welcher durch die Koeffizientenberechnungseinrichtung erhalten wurde.

8. Gerät zur Prüfung der Leberfunktion nach Anspruch 7, weiterhin umfassend eine Einrichtung (37, 38) zum Ausgeben der Rückhalterate (R), welche durch die Einrichtung zum Erhalten der Rückhalterate erhalten wurde.

9. Gerät zur Prüfung der Leberfunktion nach Anspruch 2, worin die Arithmetikeinrichtung (34) eine Einrichtung zum Berechnen des Cg-Wertes umfaßt, welcher mit der Konzentration des bestimmten Farbstoffes korreliert ist, auf der Basis der Konstante A, welche von der Einrichtung erhalten wurde in Übereinstimmung mit dem folgenden Ausdruck: 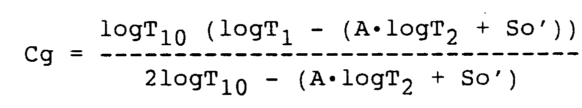 wobei T₁ und T₂ die Werte der abgetasteten ersten und zweiten photoelektrischen Konversionssignale wiedergeben.

10. Gerät zur Prüfung der Leberfunktion nach Anspruch 4, worin die Koeffizientenberechnungseinrichtung zum Berechnen der Konstanten A und B auf der Basis des folgenden Ausdruckes umfaßt: 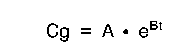 wobei t eine bestimmte Zeit nach Einspritzen des bestimmten Farbstoffes wiedergibt.

11. Gerät zur Prüfung der Leberfunktion nach Anspruch 5, worin die Einrichtung zum Erhalten der Blutplasmaschwundrate eine Einrichtung zum Ausführen der Berechnung des folgenden Ausdrucks umfaßt: 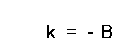 wobei k die Blutplasmaschwundrate wiedergibt.

12. Gerät zur Prüfung der Leberfunktion nach Anspruch 7, worin die Einrichtung zum Erhalten der Rückhalterate eine Einrichtung zum Ausführen der Berechnung des folgenden Ausdrucks umfaßt: 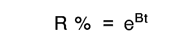 wobei R % die Rückhalterate wiedergibt.

13. Gerät zur Prüfung der Leberfunktion nach Anspruch 1, worin die Entscheidungseinrichtung eine Einrichtung (SP28) zum Berechnen eines Korrelationskoeffizienten r₁ des Regressionslinienausdruckes umfaßt.

14. Gerät zur Prüfung der Leberfunktion nach Anspruch 12, weiterhin umfassend eine Informationseinrichtung (33, SP30) zum Ausgeben eines Alarmes, wenn der Korrelationskoeffizient (r₁), welcher von der Einrichtung zum Berechnen des Korrelationskoeffizienten erhalten wurde, kleiner als ein vorbestimmter Wert ist.

15. Gerät zur Prüfung der Leberfunktion nach Anspruch 4, worin die Koeffizientenberechnungseinrichtung eine Einrichtung (SP52) zum Berechnen eines Korrelationskoeffizienten (r₂) der Simulationsfunktion umfaßt.

16. Gerät zur Prüfung der Leberfunktion nach Anspruch 15, weiterhin umfassend eine Informationseinrichtung (33, SP54) zum Ausgeben eines Alarmes, wenn der Korrelationskoeffizient (r₂) der Simulationsfunktion kleiner als ein bestimmter Wert ist.

17. Gerät zur Prüfung der Leberfunktion nach Anspruch 1, we iterhin umfassend eine Betriebsauswahleinrichtung (41, 42) zum Auswählen einer Biokalibrationsbetriebsart zum Durchführen des Vorganges für die Bestimmung des Regressionskoeffizienten des Regressionslinienausdrucks von der Entscheidungseinrichtung und einer Meßbetriebsart zum Durchführen des Vorganges der Berechnung des Wertes (Cg) welcher mit der Konzentration des bestimmten Farbstoffes korreliert ist, durch die Arithmetikeinrichtung.

18. Gerät zur Prüfung der Leberfunktion nach Anspruch 17, weiterhin umfassend eine Einrichtung (42) zum Aktivieren der Entscheidungseinrichtung in Antwort auf die Auswahl der Biokalibrationsbetriebsart durch die Betriebsartauswahleinrichtung.

19. Gerät zur Prüfung der Leberfunktion nach Anspruch 17, we iterhin umfassend eine Einrichtung zum Aktivieren der Arithmetikeinrichtung (34) in Antwort auf die Auswahl der Meßbetriebsart durch die Betriebsartauswahleinrichtung (41, 42).

20. Gerät zur Prüfung der Leberfunktion nach Anspruch 1, we iterhin umfassend eine Setzeinrichtung (SP241-SP249) zum Einstellen von Intensitätspegeln des ersten und zweiten Lichtes, welches von der Lichtquelleneinrichtung emittiert wird, so daß die Pegel der ersten und zweiten photoelektrischen Konversionssignale innerhalb eines vorbestimmten Bereiches liegen.

## Revendications

1. Appareil pour le fonctionnement du foie, destiné à tester le fonctionnement du foie, comprenant:
des moyens de source de lumière (11, 12) pour exposer le tissu vital à une première lumière dune longueur donde absorbée par un colorant spécifique dosé dans le sang dudit tissu vital et destiné à passer dans le foie et à être éliminé par lui, et une seconde lumière dune longueur donde non absorbée par ledit colorant spécifique ;
des moyens de conversion photoélectrique (13) pour produire des premiers et seconds signaux de conversion photoélectrique, correspondant à ladite première lumière et à ladite seconde lumière, appliquées audit tissu vital par lesdits moyens de source de lumière, et obtenus à partir dudit tissu vital
des moyens d'échantillonnage (28) pour échantillonner des premiers et seconds signaux de conversion photoélectrique provenant desdits moyens de conversion photoélectrique ;
des moyens de détection de pulsations (28, 28", SP51-SP54) pour détecter uniquement les composants de pulsation desdits premiers et seconds signaux de conversion photoélectrique venant desdits moyens de conversion photoélectrique ;
des moyens de décision (34, SP28) pour déterminer le coefficient de régression d'une droite de régression entre les logarithmes des intensités desdits composants de pulsation desdits premiers et seconds signaux de conversion photoélectrique, obtenus à partir desdits moyens de détection de pulsations immédiatement avant l'injection dudit colorant spécifique, et une valeur de base (50), en utilisant lesdits premiers et seconds signaux de conversion photoélectrique échantillonnés par lesdits moyens d'échantillonnage ; et
des moyens arithmétiques (34) pour calculer une valeur (Cg) en corrélation avec une concentration du colorant spécifique dans ledit sang, sur la base dune sortie desdits moyens d'échantillonnage pendant une période prescrite à partir de linjection dudit colorant spécifique et de ladite pente de la droite de régression et de ladite valeur de base (50) déterminée par lesdits moyens de décision.

2. Appareil de contrôle du fonctionnement du foie selon la revendication 1, dans lequel
lesdits moyens de détection de pulsations (28, 28", SP51-SP54) comprennent des moyens pour échantillonner, une pluralité de fois, lesdits premiers et seconds signaux de conversion photoélectrique, et lesdits moyens de décision (34, SP28), pour déterminer ladite valeur de base (50) en utilisant ledit coefficient de régression de ladite droite de répression, comprennent des moyens pour obtenir une constante A en effectuant une analyse de la droite de régression selon l'expression suivante : 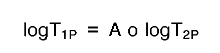 où Tₗp et T₂p représentent des valeurs moyennes desdits premiers et seconds signaux de conversion photoélectrique échantillonnés par lesdits moyens d'échantillonnage (28), une pluralité de fois.

3. Appareil de contrôle du fonctionnement du foie selon la revendication 1, dans lequel
lesdits moyens déchantillonnage (28) comprennent des moyens pour échantillonner lesdits premiers et seconds signaux de conversion photoélectrique une pluralité de fois, et des moyens pour obtenir ladite valeur de base So selon l'expression suivante : 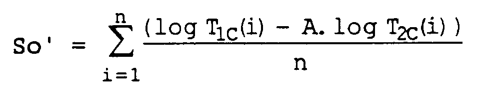 où T_{1c} et T_{2c} représentent lesdits premiers et seconds signaux de conversion photoélectrique échantillonnés ladite pluralité de fois (n fois), et obtenant une valeur maximale T₁₀ dudit premier signal de conversion photoélectrique.

4. Appareil de contrôle du fonctionnement du foie selon la revendication 1, comprenant en outre des moyens de calcul de coefficient (34, SP52) pour obtenir un coefficient (B) dune fonction de simulation, en fonction du temps, en utilisant la méthode des moindres carrés sur la base de ladite valeur (Cg) en corrélation avec ladite concentration de colorant spécifique, obtenue par lesdits moyens arithmétiques.

5. Appareil de contrôle du fonctionnement du foie selon la revendication 4, comprenant en outre des moyens (34, SP52) pour obtenir une vitesse de disparition (K = -8) dans le plasma sanguin dudit colorant spécifique sur la base dudit coefficient (B) de ladite fonction de simulation, obtenue par lesdits moyens de calcul de coefficient.

6. Appareil de contrôle du fonctionnement du foie selon la revendication 5, comprenant en outre des moyens (37, 38) pour fournir ladite vitesse de disparition (K) dans le plasma sanguin, obtenue par lesdits moyens pour obtenir ladite vitesse de disparition dans le plasma sanguin.

7. Appareil de contrôle du fonctionnement du foie selon la revendication 4, comprenant en outre des moyens (34,SP52) pour obtenir un taux de rétention (R) dudit colorant spécifique dans ledit temps prescrit sur la base dudit coefficient de ladite fonction de simulation, obtenu par ledit moyen de calcul de coefficient.

8. Appareil de contrôle du fonctionnement du foie selon la revendication 7, comprenant en outre des moyens (37, 38) pour fournir ledit taux de rétention (R) obtenu par lesdits moyens pour obtenir ledit taux de rétention.

9. Appareil de contrôle du fonctionnement du foie selon la revendication 2, dans lequel
lesdits moyens arithmétiques (34) comprennent des moyens pour calculer ladite valeur Cg en corrélation avec ladite concentration de colorant spécifique sur la base de ladite constante A, obtenue par lesdits moyens dobtention selon l'expression suivante : 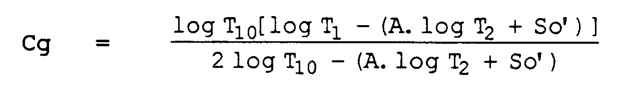 où Ti et T₂ représentent des valeurs desdits premiers et seconds signaux de conversion photoélectrique échantillonnés.

10. Appareil de contrôle du fonctionnement du foie selon la revendication 4, dans lequel
lesdits moyens de calcul de coefficient comprennent des moyens pour calculer des constantes A et B sur la base de l'expression suivante : 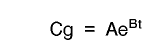 où t représente ladite période prescrite après linjection dudit colorant spécifique.

11. Appareil de contrôle du fonctionnement du foie selon la revendication 5, dans lequel
lesdits moyens pour obtenir la vitesse de disparition dans le plasma sanguin comprennent des moyens pour effectuer le calcul de l'expression suivante : 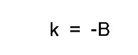 où k représente ladite vitesse de disparition dans le plasma sanguin.

12. Appareil de contrôle du fonctionnement du foie selon la revendication 7, dans lequel
lesdits moyens pour obtenir ledit taux de rétention comprennent des moyens pour effectuer le calcul de l'expression suivante : 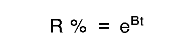 où R % représente ledit taux de rétention.

13. Appareil de contrôle du fonctionnement du foie selon la revendication 1, dans lequel
lesdits moyens de décision comprennent des moyens (SP28) pour calculer un coefficient de corrélation r, , de ladite droite de régression.

14. Appareil de contrôle du fonctionnement du foie selon la revendication 12, comprenant en outre des moyens d'information (33, SP30) pour produire une alarme lorsque ledit coefficient de corrélation (Tᵢ ), obtenu par lesdits moyens pour calculer ledit coefficient de corrélation, est inférieur à une valeur prédéterminée.

15. Appareil de contrôle du fonctionnement du foie selon la revendication 4, dans lequel
lesdits moyens de calcul de coefficient comprennent des moyens (SP52) pour calculer un coefficient de corrélation (T₂) de ladite fonction de simulation.

16. Appareil de contrôle du fonctionnement du foie selon la revendication 15, comprenant en outre des moyens d'information (33, SP54) pour produire une alarme lorsque ledit coefficient de corrélation (T₂) de ladite fonction de simulation est supérieur à une valeur prédéterminée.

17. Appareil de contrôle du fonctionnement du foie selon la revendication 1, comprenant en outre des moyens de sélection de mode (41, 42) pour sélectionner un mode de bioétalonnage pour effectuer l'opération pour déterminer ledit coefficient de régression de ladite droite de régression par lesdits moyens de décision, et un mode de mesure pour effectuer l'opération pour le calcul de ladite valeur (Cg) en corrélation avec ladite concentration de colorant spécifique, par lesdits moyens arithmétiques.

18. Appareil de contrôle du fonctionnement du foie selon la revendication 17, comprenant en outre des moyens (42) pour mettre en fonction lesdits moyens de décision en réponse à la sélection du mode de bioétalonnage par lesdits moyens de sélection de mode.

19. Appareil de contrôle du fonctionnement du foie selon la revendication 17, comprenant en outre des moyens pour mettre en fonction lesdits moyens arithmétiques (34) en réponse à la sélection dudit mode de mesure par lesdits moyens de sélection de mode (41, 42).

20. Appareil de contrôle du fonctionnement du foie selon la revendication 1, comprenant en outre des moyens de réglage (SP241-SP249) pour régler des niveaux d'intensités de ladite première lumière et de ladite seconde lumière émises par lesdits moyens de source de lumière afin que les niveaux desdits premiers et seconds signaux de conversion photoélectrique soient situés dans une plage prédéterminée.
